# EUROPEAN PATENT SPECIFICATION

(11) **EP 3 350 308 B1**
(45) Date of publication and mention of the grant of the patent: **17.02.2021**
(21) Application number: 16763834.5
(22) Date of filing: 13.09.2016
(51) Int. Cl.: C11D 17/04, C11D 1/62, A61K 8/02

(54) **USE OF A LYOCELL FIBRE**
VERWENDUNG EINER LYOCELL-FASER
UTILISATION D'UNE FIBRE LYOCELL

(30) Priority: 16.09.2015 EP 15185393
(43) Date of publication of application: 25.07.2018
(73) Proprietor: Lenzing Aktiengesellschaft, 4860 Lenzing (AT)
(72) Inventor: OPIETNIK, Martina, 4861 Schörfling a. Attersee (AT); GOLDHALM, Gisela, 3363 Neufurth (AT); FIRGO, Heinrich, 4840 Vöcklabruck (AT)
(74) Representative: Nemec, Harald
(86) International application number: PCT/EP2016/071486
(87) International publication number: WO 2017/046044

(56) References cited:
- EP-A2- 1 881 058
- WO-A1-99/47733
- WO-A1-2004/001128
- WO-A1-2004/062703
- WO-A1-2006/013378

## Description

The present invention relates to the use of a lyocell fibre in a product comprising a non-woven substrate and a liquid containing a compound with a quaternary ammonium salt. Such products are in the following also referred to as "wet or moist wipes".

Especially, the present invention relates to the use of a lyocell fibre in wet or moist wipes for hygiene products, especially for disinfecting and sanitizing wipes for home, food service, medical and other market areas.

For hygiene purposes wet or moist wipes, respectively, containing quaternary ammonium salts as active substances are commonly used. The wipes mainly consist of a non-woven substrate which is impregnated with a liquid, such as a solution or dispersion containing said quaternary ammonium salt. This liquid is in the following also referred to as "lotion". The quaternary ammonium salt exerts an antimicrobial activity.

In the US, the compounds and the amounts of active ingredient present in an antimicrobial wipe solution are specified in EPA docket numbers: EPA-HQ-OPP-2006-0339 and EPA-HQ-OPP-2006-0338 and 21 code of Federal Regulations § 178.1010 (b) and (c). The FDA regulation applies to "food processing equipment and on other food-contact article as specified", whereas the EPA regulation covers most of the other uses, primarily sanitation wipes for use with hard surfaces. § 178.1010 (b) specifies the compounds which can be used and § 178.1010 (c) specifies the amounts of the active ingredients in the solution in at least amounts and not to exceed amounts, whereas the EPA regulations specify the maximum quaternary ammonium salt concentrations.

Wet wipes which fulfill the regulations cited today mainly consist of Polyester (PES) fibres, Polypropylene (PP) fibres or mixtures thereof, as well as mixtures of these fibres with a low amount of only very few percents of cellulosic fibres.

Wet wipes are normally packed in packages with stacks of typically 10 to over 100 single pieces. During storage the lotion tends to seep from top to bottom due to gravity and poor interaction with the non-cellulosic fibres. This leads to a non-uniform distribution of the lotion which affects the effectiveness of a single wipe from the pack. Due to the hydrophobic properties of PES and PP fibres this effect is increased if aqueous formulations are employed.

To decrease this effect, cellulosic fibres may be used, as these fibres are hydrophilic and are able to improve the distribution of lotions. However, each cellulosic fibre contains anionic carboxylic groups. An interaction between the anionic carboxylic groups and the cationic substance (active substance) in the lotion reduces the effectiveness of these products.

To cope with this problem, the load of quaternary ammonium salts on the wipe can be increased in order to compensate for the chemical tie-up. However, this over-loading sometimes puts the product outside of the FDA monograph.

According to US 2011/0220311, dissolved metal salts are added to the lotion to prevent adsorption of quaternary ammonium salts by the cellulosic fibres. The salts have much smaller cations compared to the active substances in the lotion. Therefore, these cations can bind much faster to the surface of the cellulose and mask the anionic charges. As a consequence, the active substance does not bind to the cellulose and is free to work as a disinfectant.

According to US 2006/0193990, a binder compromising a polymer and a surfactant is added, which compensates the surface charge on the fibres of the web product, whereby the cationic functional agent remains free in the lotion and is deliverable to the surface in sufficient amounts for the desired disinfecting effect.

US2005/0245151 concerns a method for increasing the release of a cationic component of a lotion from a non-woven wet wipe material by adding a chemical blocking material (polyamide-epichlorhydrine resin, polyamide resin, melamine resin, high molecular weight cationic chemical material) to the non-woven fabric prior to saturation of the fabric with a lotion.

In WO 2007/016579 a cationic fibrous sanitizing substrate comprising a cationic fibrous compound, such as a chitosan fibre with a cationic finish, is disclosed.

US 7 637 271 (The Clorox Company) discloses the addition of 0,1-20% of a polyaluminium compound (chlorid, chlorohydrate, sulfate) to a cleaning composition.

In WO 2004/0062703 the use of a fibre modified with certain functions and hygiene products containig said fibres are disclosed. Said functions are especially cationic groups and aldehydes, which are capable of interacting with anionic groups and amine groups.

According to WO 2004/001128, by treating fibres of a fibrous web with a solution of a chemical blocking material prior to soaking the web with a chemical lotion containing the cationic lotion component, a fibrous web that retains less of cationic lotion components is obtained.

EP 1 881 058 discloses self-disinfecting cleaning wipes made of cellulosic fibres and modified Lyocell fibres, which comprise at least one modified polymer with acidic groups and are loaded with bactericidal metal ions.

According to the state of the art additional chemicals and after-treatment steps are necessary in order to influence the surface charge of the cellulosic fibres employed in wet and moist wipe products. This causes additional costs and chemical load.

There is a demand for wet wipes having sufficient release properties and to eliminate fluid migration in the package.

This problem underlying the present invention is solved by the use of a lyocell fibre in a product comprising a non-woven substrate and a liquid containing a compound with a quaternary ammonium salt, wherein said lyocell fibre is contained in said non-woven substrate, characterized in that said lyocell fibre contains an essentially water-insoluble salt incorporated in said fibre in an amount of from 1 wt.% to 10 wt.%, based on the weight of the dry fibre, wherein said salt has a solubility in water at 15°C to 25°C of 5 g/l or less, such as 3 g/l or less, 1 g/l or less, preferably less than 0.1 g/l, and wherein said salt is soluble under acidic conditions.

Preferred embodiments of the present invention are disclosed in the dependent claims.

### DETAILED DESCRIPTION OF THE INVENTION

The use of a lyocell fibre containing an essentially water-insoluble salt incorporated in said fibre surprisingly solves the problem of wet or moist wipes discussed above.

Especially, by way of incorporating an essentially water-insoluble salt into the lyocell fibre, it has been found that there is no need for a further treatment or addition of further materials in the stage of producing the wet or moist wipe in order to avoid the problems discussed above.

The term "lyocell" is the generic name allocated by BISFA (The International Bureau for the Standardization of man made fibres) for cellulose fibres which are produced by dissolving cellulose in an organic solvent without the formation of a derivative and extruding fibres from said solution by means of a dry-wet spinning process or a melt-blown process. In this regard, an organic solvent is understood to be a mixture of an organic chemical and water. At present, N-methyl-morpholine-N-oxide (NMMO) is used as an organic solvent on a commercial scale.

In said process, the solution of the cellulose is usually extruded by means of a forming tool, whereby it is moulded. Via an air gap, the moulded solution gets into a precipitation bath, where the moulded body is obtained by precipitating the solution. The moulded body is washed and optionally dried after further treatment steps. A process for the production of lyocell fibres is described, for instance, in US A 4,246,221. Lyocell fibres are distinguished by a high tensile strength, a high wet-modulus and a high loop strength.

Under the term "incorporated in said fibre", the skilled artisan understands that the essentially water-insoluble salt is distributed within the cellulosic matrix of the fibre essentially throughout the whole cross-section of it.

It is well-known to modify cellulosic fibres and also lyocell fibres via the incorporation of various additives.

Incorporation of a modifying compound into a lyocell fibre can be achieved, as is known per se to the skilled artisan, by admixing the compound to the spinning solution before the spinning process and/or by admixing the compound to a precursor of the solution.

As a "precursor" of the spinning solution, starting and intermediate materials for the manufacture of the spinning solution are to be understood, especially
- the starting cellulose material employed, e.g. pulp
- the solvent employed (aqueous tertiary amine oxide, in the following the term "NMMO" is used as an abbreviation for all suitable amine oxides)
- a mixture of the starting cellulose material with the solvent NMMO, especially a suspension of the cellulose in an aqueous NMMO, starting from which the solution can be made.

WO 2009/036481 discloses a lyocell fibre containing a material selected from the group consisting of pearl powder, ground nacre and mixtures thereof incorporated therein.

In the following, a lyocell fibre containing an essentially water-insoluble salt incorporated therein, as employed by the present invention, is referred to as the "modified lyocell fibre".

Under an "essentially water-insoluble" salt the skilled artisan understands a salt which has a very low solubility in water so that it is not washed out in the aqueous milieu of producing lyocell fibres including their aftertreatment, or is only washed out to an extent which is acceptable in terms of the ecological and economical requirements of a commercial production.

The salt employed in the present invention has a solubility in water at 15°C to 25°C of 5 g/l or less, such as 3 g/l or less, 1 g/l or less, especially preferred less than 0.1 g/l.

The salt is soluble under acidic conditions.

Typically, the lotion containing the compound with a quaternary ammonium salt, which is applied to a non-woven substrate in order to produce a wet or moist wipe, has a pH-value of about of smaller than 7, especially of from 3 to 6, such as of from 4.0 to 5.5.

Under these acidic conditions the salt contained in the fibre is soluble and will dissociate to a certain extent. The cation of the salt then binds to the cellulose, thus masking the anionic charges of the cellulose without the need of the addition of further additives.

As an alternative, the modified lyocell fibre, or the non-woven substrate containing the modified lyocell fibre, can be treated with an acidic medium before the treatment with the lotion containing the quaternary ammonium salts. Again by way of such treatment the anionic charges of the cellulosic fibre would be masked. This alternative would enable the use of lotions with no acidic pH-value in the production stage of the wet or moist wipe. Furthermore, more stringent treatment conditions (such as the use of stronger acids) can be applied under this alternative.

Lyocell fibres typically contain an amount of about 20 µmol to 50 µmol, especially 25 µmol to 40 µmol carboxylic, i.e. anionic groups per g of fibre. Generally spoken, therefore, the objective is to provide a modified fibre in which, upon an acidic treatment of the fibre, a sufficient amount of cations from the salt contained in the fibre will be released in order to mask these anionic charges as completely as possible.

As is readily apparent, this objective can be solved on the basis of the skilled artisan's knowledge, by appropriately selecting the parameters of
- the nature of the salt and, thus, both its valence and its solubility
- the amount of salt contained in the fibre and
- the nature and amount of the acidic liquid with which the fibres and/or the non-woven substrate are treated.

In a preferred embodiment of the present invention, the essentially water-insoluble salt employed may be selected from the group consisting of salts of alkali metals, earth alkali metals, transition metals, aluminium and mixtures thereof.

Further preferably, the salt is an inorganic salt.

Especially preferred, the salt is selected from the group consisting of calcium carbonate, zinc oxide, calcium phosphate, magnesium hydroxide, aluminium hydroxide, calcium oxalate, barium carbonate, organic oxalates and mixtures thereof.

The lyocell fibre used according to the present invention preferably contains said inorganic salt in an amount of from 2 wt.% to 7 wt.%, based on the weight of the dry fibre.

The quaternary ammonium salt employed for making the wet or moist wipe is preferably a quaternary ammonium chloride.

Especially, said quaternary ammonium salt may be selected from the group consisting of n-alkylbenzyldimethylammoniumchloride (BAC or ABDAC), di-n-alkyldimethyl ammoniumchloride (DDAC), n-alkyldimethylethylbenzylammonium chloride (ADEBAC), n-alkyltrimethylammonium chloride, octyldecyldimethylammonium chloride, didecyldimethylammonium chloride, dioctyldimethylammonium chloride, benzethonium chloride and mixtures thereof.

The non-woven substrate containing the modified lyocell fibre can be manufactured by means known to the skilled artisan. In a preferred embodiment of the present invention, the non-woven substrate containing the modified lyocell fibre is a melt-blown product. The manufacture of melt-blown products from solutions of cellulose in NMMO is known, e.g. from, *inter alia,* WO 98/26122, WO 99/47733, WO 98/07911, US 6,197,230, WO 99/64649, WO 05/106085, EP 1 358 369 and WO 2007/124521.

Preferably, the non-woven substrate contains the modified lyocell fibre in an amount of from 5 wt.% to 100 wt.%, preferably 10 wt.% to 50 wt.%, especially preferred 20 wt.% to 30 wt.%, based on the weight of the dry substrate.

Under a non-woven substrate containing the modified lyocell fibre in an amount of "100 wt.%", the skilled artisan understands a substrate that essentially consists of said modified lyocell fibre, such as a melt-blown non-woven product produced from a solution of cellulose in NMMO. The presence of minor amounts of additives, such as for surface treatment of the fibre and/or the non-woven substrate, is not excluded thereby.

As known per se, the non-woven substrate may contain a mixture of the modified lyocell fibre with hydrophobic fibres, such as PP fibres, PES fibres and mixtures thereof.

The present invention also relates to a product comprising a non-woven substrate and a solution containing a compound with a quaternary ammonium salt, wherein a lyocell fibre is contained in said non-woven substrate, characterized in that said lyocell fibre contains an essentially water-insoluble salt incorporated in said fibre in an amount of from 1 wt.% to 10 wt.%, based on the weight of the dry fibre, wherein said salt has a solubility in water at 15°C to 25°C of 5 g/l or less, such as 3 g/l or less, 1 g/l or less, preferably less than 0.1 g/l, and wherein said salt is soluble under acidic conditions.

As to the details of the fibre employed in the product of the present invention, i.e. the wet or moist wipe, the nature of the salt and other preferred embodiments, reference may be made to the above paragraphs.

### EXAMPLES:

### Example 1: Improvement of release properties of BAC by incorporation of CaCO₃

Lyocell fibres having 1.1 wt%; 2.9 wt%; 4.1 wt%; 4.8 wt% and 6.2 wt% (based on bone-dry fibre), respectively, CaCO₃ incoporated therein were produced according to the state of the art. Thus, a 13wt% spinning dope of cellulose in aqueous NMMO was prepared containing the above-named concentrations of CaCO₃. Subsequent spinning of the dope using the lyocell process led to the final fibre (1.7 dtex, 38 mm).

These air-dried fibres were mingled with 400 wt% (based on air-dried fibres) aqueous BAC-solution (alkyldimethylbenzylammonium chloride, 1000 ppm), and the release properties were determined using the titration method explained below in example 4. The aqueous BAC solution was adjusted to an acidic pH.

It becomes apparent that with increased incorporation of CaCO₃ the concentration of accessible active substance increases. In table 1 the results of the titrations of the different CaCO₃ fibre types are summarized.

**Table 1: Evaluation of titrations of the different CaCO₃ incorporated fibres.**

| Fibre type | Release of BAC [%] |
|---|---|
| 1.1% CaCO₃ | 75 |
| 2.9% CaCO₃ | 77 |
| 4.1% CaCO₃ | 80 |
| 4.8% CaCO₃ | 86 |
| 6.2% CaCO₃ | 90 |

### Example 2: Improvement of release properties of BAC by incorporation of ZnO

Lyocell fibres having 1.5 wt%; 2 wt%; 2.5 wt% und 3.0 wt% (based on bone-dry fibre), respectively, ZnO incorporated therein were produced according to the state of the art. Thus, a 13wt% spinning dope of cellulose in aqueous NMMO was prepared containing the above-named concentrations of ZnO. Subsequent spinning of the dope using the lyocell process led to the final fibre (1.7dtex, 38mm).

These air-dried fibres were mingled with 400 wt% (based on air-dried fibres) aqueous BAC-solution (alkyldimethylbenzylammonium chloride, 1000 ppm), and the release properties were determined using the titration method explained below in example 4. The aqueous BAC solution was adjusted to an acidic pH.

In table 2 the results of the titrations of the different ZnO fibre types are summarized.

**Table 2: Evaluation of titrations of the different ZnO incorporated fibres.**

| Fibre type | Release of BAC [%] |
|---|---|
| 1,5% ZnO | 85 |
| 2% ZnO | 90 |
| 2,5% ZnO | 90 |
| 3% ZnO | 92 |

### Example 3: At least twofold increase of BAC release properties by incorporation of insoluble salts

The fibre types mentioned in example 1 and 2 were compared with standard lyocell fibres not containing a water-insoluble salt regarding their BAC release properties.

It becomes apparent that the fibre types employed according to the present invention compared to the standard lyocell fibres show an at least twofold release of BAC.

The quaternary release was determined using the titration method described in example 4. In table 3, the results of the titrations of the different fibre types are summarized.

**Table 3: Titration results of different fibre types.**

| Fibre type | Release of BAC (%) |
|---|---|
| TENCEL® standard | 29 |
| 4.8% CaCO₃ | 86 |
| 2% ZnO | 90 |

### Example 4: Determination of BAC release properties using a 2-phase-titration

The cationic substance (BAC or other QACs) to be determined is titrated using sodium dodecylsulfate in a two-phase system containing water and a chlorinated solvent (dichloromethane, chloroform,...) using bromophenol blue as indicator.

Cationic substances form a complex with bromophenyl blue which is soluble in the chlorinated solvent. Titration of this complex with the anionic surfactant leads to a replacement of the indicator anion and the new formed complex passes over to the aqueous phase. At the end of the titration the released indicator colors the chlorinated phase blue / violet, whereas the aqueous phase recolors yellowish / colorless.

### Example 5: Fibre data of the fibres with improved QAC release properties.

The fibre data of the fibre types described in examples 1 and 2 was determined. Table 4 shows that the fibre data does not change with the incorporation of the mentioned salts.

**Table 4: Fibre data**

| | Incorporation [%] | Cross section [dtex] | FFk [cN/tex] | FDk [%] |
|---|---|---|---|---|
| Standard lyocell fibre | 0 | 1,7 | 30,5 | 12 |
| 4.1% CaCO₃ | 4.1 | 1.35 | 30.5 | 12.1 |
| 4.8% CaCO₃ | 4.8 | 1.84 | 28.8 | 12.9 |
| 6.2% CaCO₃ | 6.2 | 1.76 | 28 | 12.9 |
| 3% ZnO | 3 | 1.82 | 34.2 | 13.4 |
| 2.5% ZnO | 2.5 | 1.68 | 36.7 | 13 |
| 2% ZnO | 2 | 1.84 | 33 | 13.5 |
| 1.5% ZnO | 1.5 | 1.7 | 34.06 | 13.6 |

| | | | | |
|---|---|---|---|---|
| FFk:Tenacity conditioned FDk: Elongation conditioned (FDk). | | | | |

## Claims

1. The use of a lyocell fibre in a product comprising a non-woven substrate and a liquid containing a compound with a quaternary ammonium salt, wherein said lyocell fibre is contained in said non-woven substrate, **characterized in that** said lyocell fibre contains an essentially water-insoluble salt incorporated in said fibre in an amount of from 1 wt.% to 10 wt.%, based on the weight of the dry fibre, wherein said salt has a solubility in water at 15°C to 25°C of 5 g/l or less, such as 3 g/l or less, 1 g/l or less, preferably less than 0.1 g/l, and wherein said salt is soluble under acidic conditions.

2. The use according to claim 1, **characterized in that** said salt is selected from the group consisting of salts of alkali metals, earth alkali metals, transition metals, aluminium and mixtures thereof.

3. The use according to claim 1 or 2, **characterized in that** said salt is an inorganic salt.

4. The use according to claim 1, **characterized in that** said salt is selected from the group consisting of calcium carbonate, zinc oxide, calcium phosphate, magnesium hydroxide, aluminium hydroxide, calcium oxalate, barium carbonate, organic oxalates and mixtures thereof.

5. The use according to any one of the preceding claims, **characterized in that** said lyocell fibre contains said inorganic salt in an amount of from 2 wt.% to 7 wt.%, based on the weight of the dry fibre.

6. The use according to any one of the preceding claims, **characterized in that** said non-woven substrate containing said lyocell fibre is a melt-blown product.

7. The use according to any one of the preceding claims, **characterized in that** said non-woven substrate contains said lyocell fibre in an amount of from 5 wt.% to 100 wt.%, such as 10 wt.% to 80 wt.%, 20 wt.% to 60 wt.%, especially preferred 20 wt.% to 30 wt.%, based on the weight of the dry substrate.

8. A product comprising a non-woven substrate and a solution containing a compound with a quaternary ammonium salt, wherein a lyocell fibre is contained in said non-woven substrate, **characterized in that** said lyocell fibre contains an essentially water-insoluble salt incorporated in said fibre in an amount of from 1 wt.% to 10 wt.%, based on the weight of the dry fibre, wherein said salt has a solubility in water at 15°C to 25°C of 5 g/l or less, such as 3 g/l or less, 1 g/l or less, preferably less than 0.1 g/l, and wherein said salt is soluble under acidic conditions.

9. The product according to claim 8, **characterized in that** said salt is selected from the group consisting of salts of alkali metals, earth alkali metals, transition metals, aluminium and mixtures thereof.

10. The product according to claim 8 or 9, **characterized in that** said salt is an inorganic salt.

11. The product according to claim 8, **characterized in that** said salt is selected from the group consisting of calcium carbonate, zinc oxide, calcium phosphate, magnesium hydroxide, aluminium hydroxide, calcium oxalate, barium carbonate, organic oxalates and mixtures thereof.

12. The product according to any one of claims 8 to 11, **characterized in that** said lyocell fibre contains said inorganic salt in an amount of from 2 wt.% to 7 wt.%, based on the weight of the dry fibre.

13. The product according to any one of claims 8 to 12, **characterized in that** said non-woven substrate containing said lyocell fibre is a melt-blown product.

14. The product according to any one of claims 8 to 12, **characterized in that** said non-woven substrate contains said lyocell fibre in an amount of from 5 wt.% to 100 wt.%, such as 10 wt.% to 80 wt.%, 20 wt.% to 60 wt.%, especially preferred 20 wt.% to 30 wt.%, based on the weight of the dry substrate.

## Patentansprüche

1. Verwendung einer Lyocellfaser in einem Produkt, umfassend ein Vliessubstrat und eine Flüssigkeit, die eine Verbindung mit einem quaternären Ammoniumsalz enthält, wobei die Lyocellfaser in dem Vliessubstrat enthalten ist, **dadurch gekennzeichnet, dass** die Lyocellfaser ein im Wesentlichen wasserunlösliches Salz enthält, das in die Faser in einer Menge von 1 Gew.-% bis 10 Gew.-%, bezogen auf das Gewicht der trockenen Faser, eingebracht ist, wobei das Salz bei 15°C bis 25°C eine Löslichkeit in Wasser von 5 g/l oder weniger, wie z.B. 3 g/l oder weniger, 1 g/l oder weniger, bevorzugt weniger als 0,1 g/l, aufweist und wobei das Salz unter sauren Bedingungen löslich ist.

2. Verwendung gemäß Anspruch 1, **dadurch gekennzeichnet, dass** das Salz ausgewählt ist aus der Gruppe bestehend aus Salzen von Alkalimetallen, Erdalkalimetallen, Übergangsmetallen, Aluminium und Gemischen davon.

3. Verwendung gemäß Anspruch 1 oder 2, **dadurch gekennzeichnet, dass** das Salz ein anorganisches Salz ist.

4. Verwendung gemäß Anspruch 1, **dadurch gekennzeichnet, dass** das Salz ausgewählt ist aus der Gruppe bestehend aus Calciumcarbonat, Zinkoxid, Calciumphosphat, Magnesiumhydroxid, Aluminiumhydroxid, Calciumoxalat, Bariumcarbonat, organischen Oxalaten und Gemischen davon.

5. Verwendung gemäß einem der vorhergehenden Ansprüche, **dadurch gekennzeichnet, dass** die Lyocellfaser das anorganische Salz in einer Menge von 2 Gew.-% bis 7 Gew.-%, bezogen auf das Gewicht der trockenen Faser, enthält.

6. Verwendung gemäß einem der vorhergehenden Ansprüche, **dadurch gekennzeichnet, dass** das Vliessubstrat, das die Lyocellfaser enthält, ein schmelzgeblasenes Produkt ist.

7. Verwendung gemäß einem der vorhergehenden Ansprüche, **dadurch gekennzeichnet, dass** das Vliessubstrat die Lyocellfaser in einer Menge von 5 Gew.-% bis 100 Gew.-%, wie z.B. 10 Gew.-% bis 80 Gew.-%, 20 Gew.-% bis 60 Gew.-%, besonders bevorzugt 20 Gew.-% bis 30 Gew.-%, bezogen auf das Gewicht des trockenen Substrats, enthält.

8. Produkt, umfassend ein Vliessubstrat und eine Lösung, die eine Verbindung mit einem quaternären Ammoniumsalz enthält, wobei eine Lyocellfaser in dem Vliessubstrat enthalten ist, **dadurch gekennzeichnet, dass** die Lyocellfaser ein im Wesentlichen wasserunlösliches Salz enthält, das in die Faser in einer Menge von 1 Gew.-% bis 10 Gew.-%, bezogen auf das Gewicht der trockenen Faser, eingebracht ist, wobei das Salz bei 15°C bis 25°C eine Löslichkeit in Wasser von 5 g/l oder weniger, wie z.B. 3 g/l oder weniger, 1 g/l oder weniger, bevorzugt weniger als 0,1 g/l, aufweist und wobei das Salz unter sauren Bedingungen löslich ist.

9. Produkt gemäß Anspruch 8, **dadurch gekennzeichnet, dass** das Salz ausgewählt ist aus der Gruppe bestehend aus Salzen von Alkalimetallen, Erdalkalimetallen, Übergangsmetallen, Aluminium und Gemischen davon.

10. Produkt gemäß Anspruch 8 oder 9, **dadurch gekennzeichnet, dass** das Salz ein anorganisches Salz ist.

11. Produkt gemäß Anspruch 8, **dadurch gekennzeichnet, dass** das Salz ausgewählt ist aus der Gruppe bestehend aus Calciumcarbonat, Zinkoxid, Calciumphosphat, Magnesiumhydroxid, Aluminiumhydroxid, Calciumoxalat, Bariumcarbonat, organischen Oxalaten und Gemischen davon.

12. Produkt gemäß einem der Ansprüche 8 bis 11, **dadurch gekennzeichnet, dass** die Lyocellfaser das anorganische Salz in einer Menge von 2 Gew.-% bis 7 Gew.-%, bezogen auf das Gewicht der trockenen Faser, enthält.

13. Produkt gemäß einem der Ansprüche 8 bis 12, **dadurch gekennzeichnet, dass** das Vliessubstrat, das die Lyocellfaser enthält, ein schmelzgeblasenes Produkt ist.

14. Produkt gemäß einem der Ansprüche 8 bis 12, **dadurch gekennzeichnet, dass** das Vliessubstrat die Lyocellfaser in einer Menge von 5 Gew.-% bis 100 Gew.-%, wie z.B. 10 Gew.-% bis 80 Gew.-%, 20 Gew.-% bis 60 Gew.-%, besonders bevorzugt 20 Gew. % bis 30 Gew.-%, bezogen auf das Gewicht des trockenen Substrats, enthält.

## Revendications

1. Utilisation d'une fibre de lyocell dans un produit comprenant un substrat non-tissé et un liquide contenant un composé avec un sel d'ammonium quaternaire, dans laquelle ladite fibre de lyocell est contenue dans ledit substrat non-tissé, **caractérisée en ce que** ladite fibre de lyocell contenant un sel sensiblement insoluble dans l'eau incorporé dans ladite fibre en une quantité de 1 % en poids à 10 % en poids, par rapport au poids de la fibre sèche, dans laquelle ledit sel a une solubilité dans de l'eau entre 15°C et 25°C de 5 g/l ou moins, comme 3 g/l ou moins, 1 g/l ou moins, de préférence moins de 0,1 g/l, et dans laquelle ledit sel est soluble dans des conditions acides.

2. Utilisation selon la revendication 1, **caractérisée en ce que** ledit sel est choisi dans le groupe comprenant des sels de métaux alcalins, de métaux alcalino-terreux, de métaux de transition, d'aluminium et de mélanges de ceux-ci.

3. Utilisation selon la revendication 1 ou 2, **caractérisée en ce que** ledit sel est un sel inorganique.

4. Utilisation selon la revendication 1, **caractérisée en ce que** ledit sel est choisi dans le groupe comprenant le carbonate de calcium, l'oxyde de zinc, le phosphate de calcium, l'hydroxyde de magnésium, l'hydroxyde d'aluminium, l'oxalate de calcium, le carbonate de baryum, les oxalates organiques et des mélanges de ceux-ci.

5. Utilisation selon l'une quelconque des revendications précédentes, **caractérisée en ce que** ladite fibre de lyocell contient ledit sel inorganique en une quantité de 2 % en poids à 7 % en poids, sur la base du poids de la fibre sèche.

6. Utilisation selon l'une quelconque des revendications précédentes, **caractérisée en ce que** ledit substrat non-tissé contenant ladite fibre de lyocell est un produit soufflé par fusion.

7. Utilisation selon l'une quelconque des revendications précédentes, **caractérisée en ce que** ledit substrat non-tissé contient ladite fibre de lyocell en une quantité de 5 % en poids à 100 % en poids, comme de 10 % en poids à 80 % en poids, de 20 % en poids à 60 % en poids, de manière particulièrement préférée de 20 % en poids à 30 % en poids, sur la base du poids du substrat sec.

8. Produit comprenant un substrat non-tissé et une solution contenant un composé avec un sel d'ammonium quaternaire, dans lequel une fibre de lyocell est contenue dans ledit substrat non-tissé, **caractérisé en ce que** ladite fibre de lyocell contient un sel sensiblement insoluble dans l'eau incorporé dans ladite fibre en une quantité de 1 % en poids à 10 % en poids, sur la base du poids de la fibre sèche, dans lequel ledit sel a une solubilité dans de l'eau entre 15°C et 25°C de 5 g/l ou moins, comme 3 g/l ou moins, 1 g/l ou moins, de préférence moins de 0,1 g/l, et dans lequel ledit sel est soluble dans des conditions acides.

9. Produit selon la revendication 8, **caractérisé en ce que** ledit sel est choisi dans le groupe comprenant des sels de métaux alcalins, de métaux alcalino-terreux, de métaux de transition, d'aluminium et de mélanges de ceux-ci.

10. Produit selon la revendication 8 ou 9, **caractérisé en ce que** ledit sel est un sel inorganique.

11. Produit selon la revendication 8, **caractérisé en ce que** ledit sel est choisi dans le groupe comprenant le carbonate de calcium, l'oxyde de zinc, le phosphate de calcium, l'hydroxyde de magnésium, l'hydroxyde d'aluminium, l'oxalate de calcium, le carbonate de baryum, les oxalates organiques et des mélanges de ceux-ci.

12. Produit selon l'une quelconque des revendications 8 à 11, **caractérisé en ce que** ladite fibre de lyocell contient ledit sel inorganique en une quantité de 2 % en poids à 7 % en poids, sur la base du poids de la fibre sèche.

13. Produit selon l'une quelconque des revendications 8 à 12, **caractérisé en ce que** ledit substrat non-tissé contenant ladite fibre de lyocell est un produit soufflé par fusion.

14. Produit selon l'une quelconque des revendications 8 à 12, **caractérisé en ce que** ledit substrat non-tissé contient ladite fibre de lyocell en une quantité de 5 % en poids à 100 % en poids, comme de 10 % en poids à 80 % en poids, de 20 % en poids à 60 % en poids, de manière particulièrement préférée de 20 % en poids à 30 % en poids, sur la base du poids du substrat sec.
